# EUROPEAN PATENT APPLICATION

(11) **EP 1 769 730 A1**
(43) Date of publication of application: **04.04.2007**
(21) Application number: 05765235.6
(22) Date of filing: 01.07.2005
(51) Int. Cl.: A61B 1/06, G02B 23/26

(54) **LIGHT SOURCE DEVICE AND FLUORESCENCE OBSERVATION SYSTEM**

(30) Priority: 06.07.2004 JP 2004199752; 28.12.2004 JP 2004381722
(71) Applicant: Olympus Corporation, Tokyo 151-0072 (JP)
(72) Inventor: HANDA, Keiji c/o OLYMPUS INT.PROP. SERV.CO.,LTD., Tokyo 1928512 (JP)
(74) Representative: von Hellfeld, Axel
(86) International application number: PCT/JP2005/012202
(87) International publication number: WO 2006/004038

(57) **Abstract**

A light source device is provided which suppresses intensity of background light and generates excitation light so that fluorescent observation with adequate brightness can be made.

In an electronic endoscope, a fluorescent observation filter 30 cutting off most of the excitation light and transmitting a fluorescent wavelength is provided before an image pickup device, and at the fluorescent observation, the excitation light is supplied from the light source device via an excitation-light irradiation filter 23B. This excitation light is transmitted in a narrow wavelength band in the vicinity of the cut-off wavelength in the light transmission characteristic of the fluorescent observation filter 30 and used as the background light, but its transmission light amount is limited to 2% or less of the total excitation light.

## Description

### Technical Field

The present invention relates to a light source device for fluorescent observation and a fluorescent observation system.

### Background Art

An endoscope device has been widely used in the medical and industrial fields in recent years. The endoscope device enables diagnosis of a target portion to be inspected such as an affected area in a body cavity by inserting an elongated endoscope insertion portion into the body cavity as well as therapeutic treatments by inserting a treatment instrument as necessary without requiring incision.

In the endoscope device, a subject image of the target portion to be inspected is captured into an objective optical system from a tip end portion of the endoscope insertion portion and an image is formed on an image pickup device such as CCD (Charge Coupled Device) through or without an image guide or the like. A signal photoelectrically converted at the image pickup device is signal-processed at a camera control unit (hereinafter abbreviated as CCU) and an endoscopic image is displayed on a display surface of a monitor.

There are endoscope devices which enables fluorescent observation by emitting excitation light in addition to a usual endoscopic observation using visible light.

As a conventional example of such an endoscope device which enables fluorescent observation, an endoscope device is proposed with the gist that as long as a wavelength range of a portion where a wavelength range illuminating a subject by an illumination system and an image capturing wavelength range by an image capturing system overlap each other is within 50 nm at the maximum, the image capturing system is set to a spectral transmission of 5% or more (See PCT Japanese Translation Patent Publication No. 11-511369, for example).

However, the intensity of the fluorescent light emitted from the observation target portion is extremely weak in general, and if the spectral transmission is set at the above-mentioned overlapping portion, a fluorescent image submerges due to background light by the excitation light (hereinafter also called as background illumination light or background light) with respect to the fluorescent light, and there is a possibility that the fluorescent image can not be observed with adequate brightness.

Also, various researches have been made on so-called PDD (Photo Dynamics Diagnosis). This PDD is a technology to diagnose a tumor portion that a fluorescent agent which is easily accumulated in a tumor tissue is delivered to a subject, and a fluorescent image emitted from the fluorescent agent accumulated in the tumor tissue is observed by irradiating excitation light through observation of presence or the shape of the fluorescent image.

In order to enhance contrast between the fluorescent light of the fluorescent agent accumulated in the tumor tissue and reflected light when the excitation light is irradiated to a normal tissue, such a fluorescent observation system is proposed that a filter which transmits the light in the fluorescent excitation wavelength region and cuts off the light in a high wavelength region is provided on a light source side and a filter which transmits only a part of reflected light and cuts off the light of a low wavelength region is provided on the image capturing means side (See PCT Japanese Translation Patent Publication No. 11-511369, for example).

In this proposal, an overlapping portion between the transmission characteristic of the filter on the light source side and the transmission characteristic of the filter on the image capturing means side is detected as the reflected light from the normal tissue. However, in the detected reflected light, brightness and hue are changed by variation of the transmission characteristic of the filter, and there is a problem that a desired contrast can not be obtained.

The present invention was made in view of the above point and has an object to provide a light source device which suppresses intensity of background light and generates excitation light so as to enable fluorescent observation with adequate brightness.

Moreover, the present invention has an object to provide a fluorescent observation system which can detect reflected light with stable brightness and hue all the time irrespective of variation of the transmission characteristic of the filter and improve contrast between fluorescent light and reflected light.

### Disclosure of Invention

A light source device of the present invention is a light source device which can selectively supply visible light and excitation light for fluorescent observation to an endoscope provided with an excitation light cut filter which cuts off the excitation light, in which excitation light generating means for generating excitation light is provided so that an excitation light transmission wavelength band for background illumination transmitting the excitation light is formed in a transmission wavelength region in the excitation light cut filter and a transmission light amount of the excitation light in the excitation light transmission wavelength band is more than 0% and not more than 2% of the total light amount of the excitation light.

Also, a fluorescent observation system of the present invention is a fluorescent observation system for displaying a fluorescent image which can be obtained by irradiating the excitation light to a subject containing a fluorescent substance, comprising a light source for generating illumination light including the excitation light, filter means arranged between the light source and the subject for alternately transmitting the excitation light and background light of a wavelength band separated from the excitation light for the illumination light emitted from the light source, image capturing means for capturing a fluorescent image of the subject obtained by the excitation light and a background light image of the subject obtained by the background light, and image processing means for synchronizing the fluorescent image and the background light image, giving image processing and outputting to be displayed on image display means as a fluorescent image.

### Brief Description of the Drawings

Fig. 1 is a configuration diagram showing an entire configuration of an endoscope device provided with a light source device according to a first embodiment of the present invention.
Fig. 2 is a front view showing a configuration of a filter of a rotating plate in the light source device.
Fig. 3 is a characteristic graph showing a transmission characteristic of a filter for excitation light irradiation together with a transmission characteristic of a filter for fluorescent observation.
Fig. 4 is a characteristic graph showing a transmission characteristic of an optical filter provided at an image pickup device.
Fig. 5 is an explanatory view showing a use example for explanation of an operation.
Fig. 6 is a characteristic graph showing a transmission characteristic of a filter for excitation light irradiation and a transmission characteristic of a filter for fluorescent observation according to a second embodiment of the present invention.
Fig. 7 is a characteristic graph showing a transmission characteristic of a filter for excitation light irradiation and a transmission characteristic of a filter for fluorescent observation of a variation according to the second embodiment.
Fig. 8 is a block diagram showing an entire configuration of a fluorescent observation system 51 according to a third embodiment of the present invention.
Fig. 9 is a schematic diagram showing a structure of a rotating filter 65.
Fig. 10 is a graph showing an example of a transmission characteristic of a first filter pair 67.
Fig. 11 is a graph showing an example of a transmission characteristic of a second filter pair 68 and an excitation light cut filter 24.

### Best Mode for Carrying Out the Invention

Embodiments of the present invention will be described below referring to the attached drawings.

### (First embodiment)

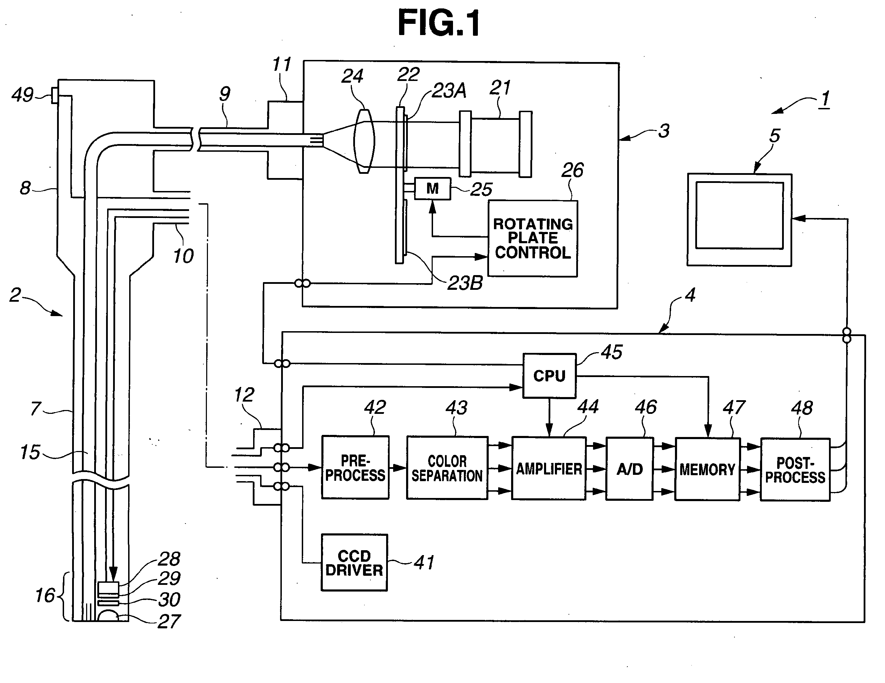

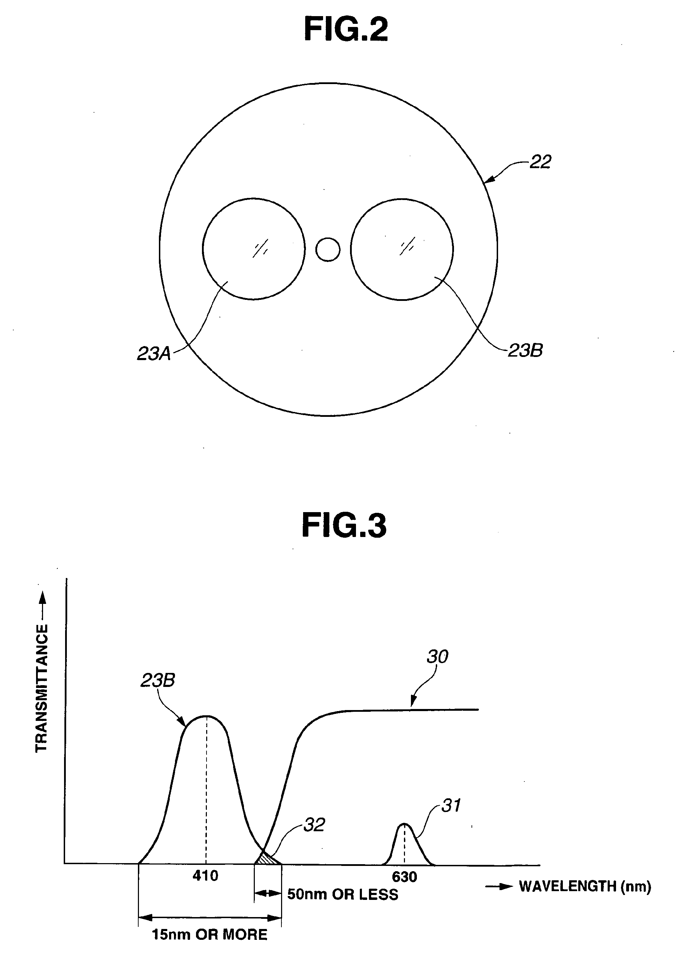

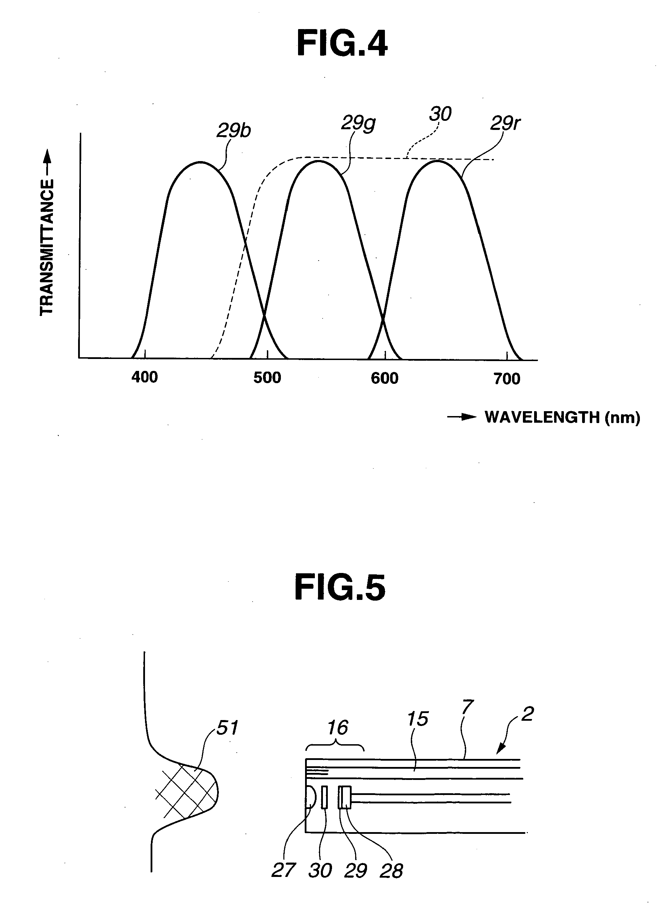

Figs. 1 to 5 relate to a first embodiment of the present invention. Fig. 1 is an entire configuration of an endoscope device provided with a light source device according to a first embodiment of the present invention. Fig. 2 is a configuration of a filter of a rotating plate in the light source device. Fig. 3 is a characteristic graph showing a transmission characteristic of a filter for excitation light irradiation together with a transmission characteristic of a filter for fluorescent observation. Fig. 4 is a characteristic graph showing a transmission characteristic of an optical filter provided at an image pickup device. Fig. 5 is an explanatory view showing a use example for explanation of an operation in a first embodiment of the present invention.

As shown in Fig. 1, the endoscope device 1 for special light observation provided with the light source device according to the present embodiment comprises an electronic endoscope 2 to be inserted into a body cavity, a light source device 3 for supplying illumination light to this electronic endoscope 2, a camera control unit (hereinafter abbreviated as CCU) 4 for signal processing for image capturing means incorporated in the electronic endoscope 2, and a monitor 5 for displaying an endoscopic image captured by the image capturing means upon input of a standard video signal outputted from this CCU 4.

The electronic endoscope 2 has an elongated insertion portion 7 and an operation portion 8 also functioning as a gripping portion provided at the rear end of this operation portion 8. A light guide cable 9 and a signal cable 10 extend from this insertion portion 7, and a light guide connector 11 and a signal connector 12 at its terminal end are connected to the light source device 3 and the CCU 4, respectively. It may be such a structure that the light guide cable 9 and the signal cable 10 are detachable from the electronic endoscope 2.

A light guide 15 inserted into the insertion portion 11 and transmitting illumination light is inserted through the light guide cable 9 extending from the operation portion 8, and by connecting the light guide connector 11 to the light source device 3, the illumination light is supplied from the light source device 3.

In the light source device 3, a lamp 21 such as a xenon lamp lighted by a lighting power supply powered from a lamp lighting circuit, not shown, is provided, and this lamp 21 generates light in a visible range as the illumination light.

The light passes through one of filters 23A, 23B mounted to a rotating plate (or turret plate) 22 and arranged on an illumination light path as shown in Fig. 2, collected by a collecting lens 24 and enters an incident end face of the light guide 15. The light having entered the incident end face is transmitted to a tip end side of the insertion portion 11 by this light guide 15. That is, it is transmitted to a light guide tip end face mounted at an illumination window of a tip end portion 16 of the insertion portion 11. And it is emitted from the tip end face and illuminates a subject such as an affected area.

This rotating plate 22 is rotatably held by a motor 25, and by a motor control circuit (or a rotating plate control circuit) 26 controlling driving of the motor 25, a filter to be arranged on the illumination light path is selected and set.

On the rotating plate 22, as shown in Fig. 2, a visible-light irradiation filter 23A for transmitting visible light and an excitation-light irradiation filter 23B for irradiating excitation light are mounted at two windows provided in the rotating direction in a light shielding disk, for example.

The visible-light irradiation filter 23A is a filter for ordinary light observation, which transmits a visible-light wavelength region of approximately 380 to 700 nm. Also, the excitation-light irradiation filter 23B is a filter for excitation light having a peak value according to the wavelength of the excitation light and used for fluorescent observation, which transmits light with the transmission wavelength width including the peak value of 15 nm or more.

This excitation-light irradiation filter 23B has its transmission peaked at 410 nm, for example, as shown in Fig. 3, more specifically, and its transmission wavelength region is 15 nm or more and set according to the characteristic of a fluorescent observation filter 30 functioning as an excitation light cut filter on the electronic endoscope 2 side, which will be described later.

At an observation window adjacent to the illumination window at the tip end portion 16 of the insertion portion 11, an objective lens 27 is mounted and forms an optical image of a subject such as an illuminated affected portion or the like. At the image forming position, a charge coupled device (hereinafter abbreviated as CCD) 28, for example, is arranged as an image pickup device, and a formed optical image is photoelectrically converted by the CCD 28.

On an image capturing face of this CCD 28, a color mosaic filter 29, for example, is arranged as an optical filter for optical color separation to enable color image capturing in the case of the visible-light illumination light.

Also, between the image capturing face of the CCD 28 and the objective lens 27, a fluorescent observation filter (or excitation light cut filter) 30 is arranged for fluorescent observation by cutting off the excitation light and transmitting a fluorescent wavelength band in the case of fluorescent observation. The spectral characteristic of this fluorescent observation filter 30 is shown in Fig. 3.

This fluorescent observation filter 30 is a filter with a transmission region which does not include a wavelength of the excitation light peak but includes a fluorescent wavelength region (shown by reference numeral 31 in Fig. 3) and a part of the wavelength region on the periphery side of the peak wavelength by the excitation light.

That is, when a wavelength region only of fluorescent light is made as a transmission region, the shape and the like is hard to be grasped. Thus, a part of a weak light intensity different from the fluorescent wavelength region 31 in the excitation light is transmitted and used as background light.

The wavelength band of the background light in this case is formed by an overlapping portion 32 where a skirt portion on the long wavelength side in the vicinity of a cutoff wavelength in the excitation-light irradiation filter 23B is overlapped with a skirt portion on the short wavelength side of the fluorescent observation filter 30.

The wavelength width of this overlapping portion 32 is more than 0 and not more than 50 nm, for example, and the total transmission light amount at the overlapping portion 32 is set to 2% or less of the total transmission light amount of the excitation light emitted through the excitation-light irradiation filter 23B.

In the present embodiment, since the fluorescent observation filter 30 having a function as an excitation light cut filter provided at the image capturing means of the electronic endoscope 2 is set to transmit only a light amount of 2% or less of the excitation light irradiated by the excitation-light irradiation filter 23B, the intensity of the background light in the case of fluorescent observation by the fluorescent observation filter 30 is effectively prevented from becoming too large. Also, in the present embodiment, the wavelength band transmitting the excitation light is set more than 0 and not more than 50 nm, for example, so that the wavelength band side for fluorescent observation is not affected.

In other words, the light source device 3 is provided with excitation light generating means which generates the excitation light such that an excitation-light transmission wavelength band for background illumination transmitting the excitation light of more than 0 and not more than 50 nm is formed in the vicinity of the cutoff wavelength in the transmission wavelength region in the excitation light cutoff filter provided on the image capturing side and the transmission light amount of the excitation light in the excitation-light transmission wavelength band is 2% or less of the total light amount of the excitation light.

In this case, the excitation light with the above characteristic is generated in the light source device 3, but the characteristic of the fluorescent observation filter 30 of the electronic endoscope 2 may be set as above with respect to the generated excitation light.

In the electronic endoscope 2 according to the present embodiment, in the illumination state of the visible light, the color mosaic filter 29 mounted to the CCD 28 to enable color image capturing comprises R, G, B filters 29r, 29g, 29b transmitting each wavelength of R, G, B as shown in Fig. 4, for example. In this case, with regard to the B filter 29b, its synthetic transmission band is narrowed to a part of the long wavelength side by the fluorescent observation filter 30 as shown in Fig. 4.

Therefore, in the present embodiment, in the ordinary observation mode at irradiation of the visible light, such control is performed that a gain for a B color signal is increased, as will be described later.

The CCD 28 is connected to a signal line inserted through the electronic endoscope 2, and by connecting the signal connector 12 to the CCU 4, a CCD drive signal is applied to the CCD 28 from a CCD driver 41 provided in the CCU 4.

An image capturing signal photoelectrically converted by the CCD 28 is inputted to a pre-process circuit 42 in the CCU 4. And after being amplified, correlated double sampling processing or the like is executed, a base-band signal component is extracted from the image capturing signal and then, it is inputted to a color separation circuit 43 and separated to R, G, B color signals by this color separation circuit 43.

An output signal of the color separation circuit 43 is inputted to an amplifier portion 44. This amplifier portion 44 can be set to a gain larger than the other color signals for the B color signal in the ordinary observation mode. Gain control of the amplifier portion 44 is executed by a CPU 45.

An output signal of the amplifier portion 44 is inputted to an A/D conversion circuit 46 and converted to a digital color signal by this A/D conversion circuit 46.

The color signal is inputted to a memory 47, and image data of the color component is temporarily stored in this memory 47. Writing/reading operation of the memory 47 is controlled by the CPU 45.

The signal read out of the memory 47 is outputted to the monitor 5 through a post-process circuit 48.

Also, an observation mode switch 49, for example, is provided at the operation portion 8 of the electronic endoscope 2, and an operation signal by this observation mode switch 49 is inputted to the CPU 45.

When the ordinary observation is set, for example, the CPU 45 controls the rotating plate control circuit 26 so that the light source device 3 supplies visible light to the light guide 15. In this case, the rotating plate control circuit 26 controls a rotating amount of the motor 25 by a control signal from the CPU 45 so that the visible-light irradiation filter 23A is arranged in the illumination light path.

On the other hand, in the ordinary observation mode, if a user such as an operator operates the observation mode switch 49 to switch to the fluorescent observation mode, the operation signal is inputted to the CPU 45. And the CPU 45 controls the rotating plate control circuit 26, and the rotating plate control circuit 26 controls the rotating amount of the motor 25 by the control signal from the CPU 45 so that the excitation-light irradiation filter 23B is arranged in the illumination light path.

The electronic endoscope 2 has a channel 50 enabling insertion of a treatment instrument as shown in Fig. 5, and a treatment such as incision of a lesion issue can be performed by inserting the treatment instrument.

Action of the endoscope device 1 of this configuration will be described using a case of fluorescent observation by delivering an agent to a lesion, for example, as an example.

As shown in Fig. 5, when a cancer tissue of bladder cancer as a tumor portion 51, for example, is to be inspected and treated, an operator has an aqueous solution of 5-ALA (5-Aminolevulinic acid) reserved in a bladder for a couple of hours, for example.

Then, the 5-ALA is changed to Protoporphyrin IX after delivery and accumulated in the tumor portion 51. Then, while this Protoporphyrin IX is being accumulated in the tumor portion 51 in this way, a treatment to detach the tumor portion 51 is performed using a resectoscope provided with an electrode for incision or an operation handle, not shown in Fig. 5, for example.

In this case, the electronic endoscope 2 is used as an optical visual tube (or video optical visual tube) of the resectoscope and under observation by the electronic endoscope 2 as shown in Fig. 5, the tumor portion 51 is incised by operating the electrode for incision, not shown.

For this incision, the ordinary observation mode is switched to the fluorescent observation mode. Then, under control of the CPU 45 as mentioned above, arrangement is switched from the visible-light irradiation filter 23A to the excitation-light irradiation filter 23B in the illumination light path of the light source device 3 and the state is brought into the fluorescent observation mode.

In this state, the light transmitted through the excitation-light irradiation filter 23B becomes the excitation light of the peak wavelength with its peak transmission at 410 nm, and this excitation light is irradiated to the vicinity of the tumor portion 51.

When the 5-ALA is delivered to induce chemical change and the tumor is observed in the Protoporphyrin IX state, the portion generates fluorescent light at 630 nm.

In the light source device 3 of the present embodiment, the excitation light is constituted to be generated such that a light amount of the excitation light transmitted through the fluorescent observation filter 30 provided at the image capturing means of the electronic endoscope 2 and entering the CCD 28 is 2% or less (but more than 0) of the total excitation light. Therefore, such a phenomenon that contrast and the like of a fluorescent image captured by fluorescent light is blurred by intensity of the background light can be effectively prevented, and a fluorescent image which is easy to be observed can be obtained.

Also, according to the present embodiment, a fluorescent image which is easy to be observed can be obtained and at the same time, a profile and the like of the tumor portion 51 and the like can be grasped by the background light. Therefore, operation to incise the tumor portion 51 can be performed smoothly.

Particularly, if color separation of the fluorescent wavelength region 31 and the overlapping portion 32 is impossible, fluorescent image information easily submerges in the conventional example due to the background light. However, according to the present embodiment, since the light amount of the background light by the overlapping portion 32 is suppressed, submerging is reduced even in that case and a fluorescent image which is easier to be observed can be obtained.

If the wavelength bands of the fluorescent wavelength region 31 and the overlapping portion 32 are different to some degree, the both can be optically color-separated by the color mosaic filter 29 as the color separation filter. Therefore, at the CCU 4, signal processing to display the both in different colors or a processing to synthesize them in the same color to be displayed can be executed.

That is, such display is made possible that the fluorescent image and the background image by the background light can be easily identified by a user or the profile of the tumor portion 51 and the like can be easily identified by synthesizing.

If the operator determines that the tumor portion 51 generating the fluorescent light is observed above the level requiring incision, for example, in the fluorescent observation, the operator can proceed with a treatment to incise the tumor portion 51.

In this case, the mode is switched to the ordinary observation mode, and the operator can insert a treatment instrument for incision into the channel 50 and perform the treatment to incise the tumor portion 51 by the treatment instrument.

As mentioned above, according to the light source device 3 of the present embodiment, excessively large intensity of the background light can be prevented and the excitation light suitable for the fluorescent observation can be generated.

Also, according to the endoscope device 1 of the present embodiment, the intensity of the background light does not become too large but a fluorescent image suitable for observation can be obtained, and a function to grasp a profile or the like by the background image by the excitation light can be ensured.

That is, according to the endoscope device 1 of the present embodiment, balance between the fluorescent light in the fluorescent observation and the background light by the excitation light can be ensured more appropriately.

In the above description, the case of the electronic endoscope 2 as the endoscope was described, but it can be also applied to a TV camera attachment type endoscope to which a TV camera incorporating image capturing means at an ocular portion of an optical endoscope is attached. In this case, the same effect can be obtained.

Also, the electronic endoscope 2 may be either a flexible endoscope with the flexible insertion portion 7 or a rigid endoscope with the rigid one. Similarly, in the case of the TV camera attachment type endoscope, the optical endoscope may be either the flexible endoscope with the flexible insertion portion 7 or the rigid endoscope with the rigid one.

In the present embodiment, since the excitation light generating means is formed for generating the excitation light such that the transmission light amount of the excitation light in the excitation light transmission wavelength band is 2% or less of the total light amount of the excitation light, the wavelength width of the overlapping portion 32 may be simply more than 0 nm, without limiting it to more than 0 and not more than 50 nm.

### (Second embodiment)

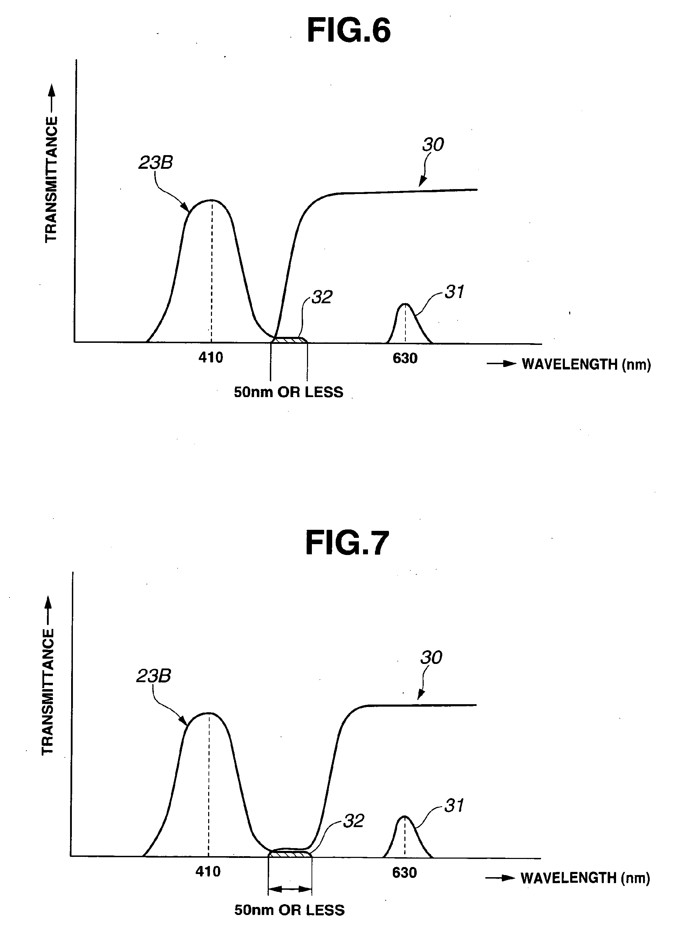

Next, a second embodiment of the present invention will be described below referring to Fig. 6. The endoscope device provided with the present embodiment has the same configuration as that in Fig. 1, and the excitation-light irradiation filter 23B built in the light source device 3 has a characteristic partially different from the characteristic shown in Fig. 3.

That is, the excitation-light irradiation filter 23B in the light source device of the present embodiment has the characteristic set as shown in Fig. 6.

This excitation-light irradiation filter 23B has the same peak wavelength with the highest transmission as that of the first embodiment, but the characteristic of a skirt portion on the long wavelength side is different from that in the first embodiment. That is, the transmission of a portion to be used as the background light is small and the transmission wavelength characteristic is a broad wavelength band.

In this case, too, the wavelength band in the overlapping portion 32 overlapping with the skirt portion of the fluorescent observation filter 30 on the short wavelength side is within 50 nm, and the transmission light amount by the overlapping portion 32 is 2% or less of the total light amount of the excitation light.

The other configurations are substantially the same as those of the first embodiment. Also, the present embodiment has the same action as that of the first embodiment.

Moreover, in the present embodiment, even if there is a variation in manufacturing characteristics between the excitation-light irradiation filter 23B and the fluorescent observation filter 30, the transmission characteristic of the excitation-light irradiation filter 23B at the overlapping portion 32 with the fluorescent observation filter 30 is small and extends over the broad wavelength region, an influence on the light amount of the background light can be reduced.

As a variation in the present embodiment, as shown in Fig. 7, the transmission characteristic at the skirt portion on the short wavelength side in the fluorescent observation filter 30 may be constituted so that the transmission characteristic is small as in the case of the excitation-light irradiation filter 23B as shown in Fig. 6 and extends over a broad wavelength band.

In this case, even if there is more variation in manufacturing characteristics than in the present embodiment between the excitation-light irradiation filter 23B and the fluorescent observation filter 30, an influence on the light amount of the background light can be reduced.

That is, according to the present embodiment and its variation, even if there is a variation in manufacturing characteristics between the excitation-light irradiation filter 23B and the fluorescent observation filter 30, an influence on the light amount of the background light can be reduced.

### (Third embodiment)

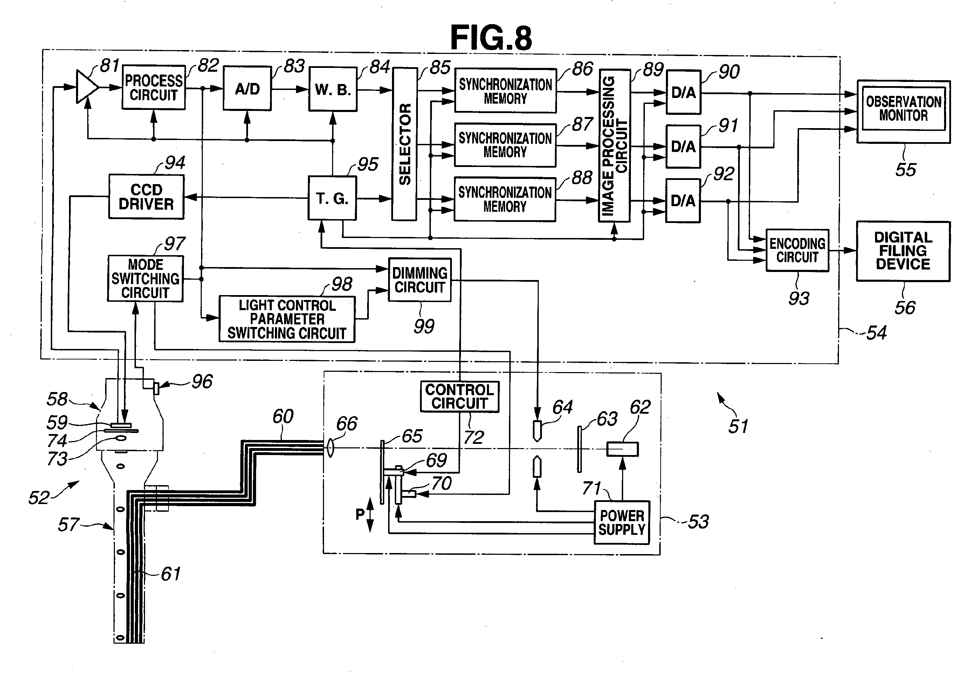

First, the entire configuration of a fluorescent observation system 51 according to a third embodiment of the present invention will be described based on Fig. 8. Fig. 8 is a block diagram for explaining the entire configuration of the fluorescent observation system 51 according to a third embodiment. As shown in Fig. 8, the fluorescent observation system 51 of the present embodiment comprises an electronic endoscope 52 provided with image capturing means, a light source device 53 for supplying illumination light to the electronic endoscope 52, a video processor 54 as image processing means for generating an image signal through signal processing of an image capturing signal outputted from the electronic endoscope 52, an observation monitor 55 as image display means for displaying an image signal outputted from the video processor 54, and an image filing device 56 for encoding the image signal outputted from the video processor 54 and storing it as a compressed image.

The electronic endoscope 52 comprises a rigid endoscope 57 to be inserted into a body cavity and a camera head portion 58 detachably connected at the base end side of the rigid endoscope 57. At the camera head portion 58, a CCD 59 as image capturing means for capturing light from a subject received by an objective lens, not shown, of the rigid endoscope 57 and generating an image capturing signal is provided. Also, a light guide cable 60 is extended from the side end face of the rigid endoscope 57, and its one end is connected to the light source device 53. In the electronic endoscope 52, a light guide 61, which is a glass fiber bundle for transmitting the illumination light to the tip end of the rigid endoscope 57 is arranged. The light guide 61 is inserted through the rigid endoscope 57, the light guide cable 60 and connected to the light source device 53 so that the illumination light emitted from the light source device 53 is guided to the tip end of the rigid endoscope 57.

The light source device 53 has a xenon lamp 62 for generating white light, which is the illumination light. The white light emitted from the xenon lamp 62 enters a disk-shaped rotating filter 65 as filter means via a diaphragm device 64 limiting the light amount after a heat ray is cut off by being transmitted through the heat-ray cut filter 63 arranged in the light path. The illumination light having made into a field sequential light by being transmitted through the rotating filter 65 is collected by a collecting lens 66 and made to enter an incident end of the light guide 61.

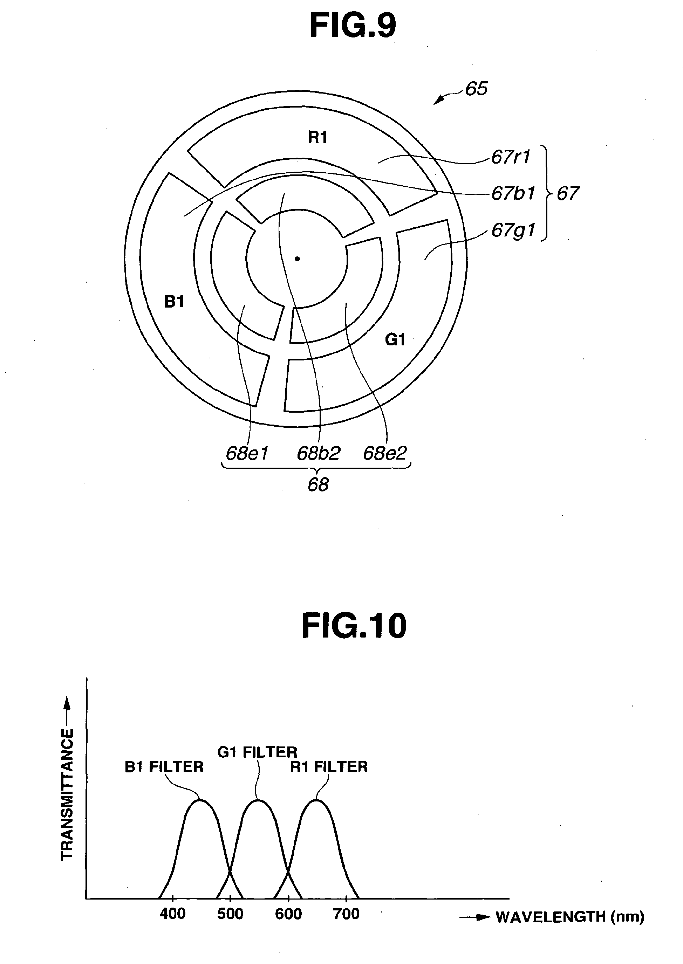

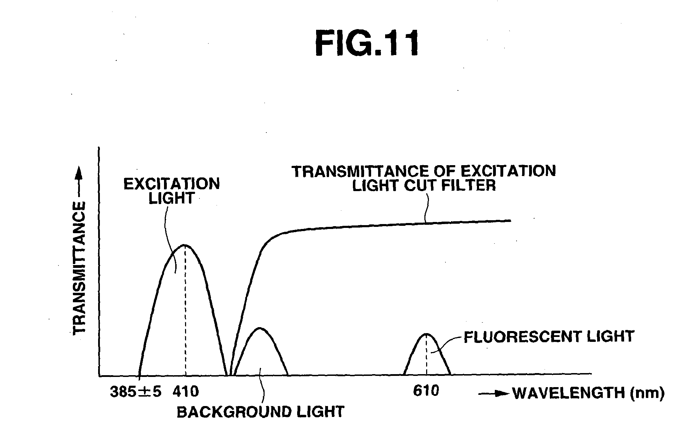

Here, the structure and characteristic of the rotating filter 65 will be described using Figs. 9 to 11. Fig. 9 is a schematic view for explaining the structure of the rotating filter 65, Fig. 10 is a diagram for explaining an example of the transmission characteristic of a first filter pair 67, and Fig. 11 is a diagram for explaining an example of the transmission characteristic of a second filter pair 68 and an excitation light cut filter 74. As shown in Fig. 9, the rotating filter 65 has the first filter pair 67 for ordinary observation arranged on the outer circumference side of a coaxial circle and the second filter pair 68 for the fluorescent observation on the inner circumference side of the coaxial circle, and either one of the filters is selected according to the observation mode, and inserted onto the light path of the illumination light. The first filter pair 67 for ordinary observation is comprised by an R1 filter 67r1, a G1 filter 67g1 and a B1 filter 67b1, and each filter has transmission characteristics as shown in Fig. 10, for example. That is, they are set so that the R1 filter 67r1 transmits a red wavelength band of 580 to 720 nm, the G1 filter 67g1 for a green wavelength band of 480 to 620 nm and the B1 filter 67b1 for a blue wavelength band of 380 to 520 nm, respectively. By being transmitted through the first filter pair 67, the illumination light becomes a field sequential light suitable for color reproduction.

On the other hand, the second filter pair 68 for the fluorescent observation is comprised by two excitation filters 68e1, 68e2 generating the excitation light and a background filter 68b2 generating the background light, and each filter has the transmission characteristic as shown in Fig. 11, for example. That is, they are set so that the excitation filters 68e1, 68e2 transmit a wavelength band of 385 to 435 nm around 410 nm, and the background filter 68b2 transmits a wavelength band of 475 to 525 nm, respectively. By being transmitted through the second filter pair 68 having such discrete spectral characteristic, the illumination light is made into the field sequential light of two narrow bands.

The rotating filter 65 having the above configuration and the characteristics is moved by a mode switching motor 70 together with a rotating filter motor 69 rotating the rotating filter 65 around the optical axis of the illumination light in the direction crossing the light path of the illumination light (direction shown by an arrow P in Fig. 8), and the either one of the first filter pair 67 and the second filter pair 68 arranged in the rotating filter 65 is selectively inserted onto the light path. Moreover, the light source device 53 is provided with a power supply 71 for supplying electric power for driving the xenon lamp 62, the diaphragm device 64, the rotating filter motor 69 and the mode switching motor 70 and a control circuit 72 for controlling operation of the rotating filter motor 69.

The illumination light having entered the light guide 61 passes through an illumination window, not shown, at the tip end face of the rigid endoscope 57 and is irradiated to a subject. In the ordinary observation mode, the illumination light of the field sequential of red (R), green (G), blue (B) is irradiated to the subject, while in the fluorescent observation mode, the illumination light of the field sequential of the excitation light and the background light is irradiated to the subject.

On the other hand, an objective optical system 73 for collecting light from the subject is provided at the camera head portion 58, and the CCD 59 is arranged at an image forming position of the objective optical system 73. At the tip end side of the CCD 59, the excitation light cut filter 74 is inserted between it and the objective optical system 73 to cut off the excitation light of 385 to 435 nm in reflected light from the subject and extract fluorescent light. The excitation light cut filter 74 is, as shown in Fig. 11, set so that the wavelength band at 470 nm and above is transmitted and set so that it does not overlap the transmission characteristic of the excitation filters 68e1, 68e2. That is, the light transmitted through the background filter 68b2 can be also transmitted through the excitation light cut filter 74 but the light transmitted through the excitation filters 68e1, 68e2 is cut off by the excitation light cut filter 74. An image capturing signal corresponding to the irradiation light having passed the respective filters of the rotating filter 65 is sequentially outputted in a time series from the CCD 59 to the video processor 54. The image capturing signal outputted in a time series becomes color signals of R, B, G in the ordinary observation mode and in the fluorescent observation mode, it becomes a fluorescent signal captured under the excitation light and a background signal captured under the background light.

The time-series image capturing signal photoelectrically converted and outputted from the CCD 59 is inputted to the video processor 54 and inputted to an amplifier 81 to be amplified to an electric signal in a predetermined range (0 to 1 volt, for example). This output signal of the amplifier 81 is inputted to a process circuit 82 for processing such as correlated double sampling or noise elimination.

The image capturing signal outputted from the process circuit 82 is inputted to an A/D converter 83 and converted into a digitalized image signal. The digital image signal outputted from the A/D converter 83 is inputted to a white balance circuit (shown as W.B. in Fig. 8) 84, and gain control is carried out for each color signal so that the level of each color signal of R, G, B becomes equal when a white subject to be a reference is captured for white balance control, that is, in order to correct variation in color tone generated from variation in optical transmission characteristics among equipments (including difference among equipments and individual differences).

The image signal outputted from the white balance circuit 84 is inputted to a 1-input/3-output selector 85. The image signal sent in the time series is separated by the selector 85 into each color signal of R, G, B or a fluorescent signal by the excitation light transmitted through the excitation filter 68e1, a fluorescent signal by the excitation light transmitted through the excitation filter 68e2 and a background light signal, sequentially inputted to synchronization memories 86, 87, 88, and synchronized at reading out. Each signal outputted from the synchronization memories 86, 87, 88 is inputted into an image processing circuit 89 for gamma correction processing, edge enhancement processing, color tone control processing and the like.

Each signal outputted from the image processing circuit 89 is inputted to D/A converting circuits 90, 91, 92, respectively and converted to an analog signal and outputted to the observation monitor 55 and an encoding circuit 93. On the observation monitor 55, an endoscopic image illuminated and captured by the ordinary white light or a fluorescent image illuminated and captured by the excitation light and the background light are displayed. The analog signal converted by the D/A converting circuits 90, 91, 92 is also outputted to the encoding circuit 43 other than to the observation monitor 55 and encoded. The encoded signal is outputted to the digital filing device 56 for filing images and stored as a compressed image.

At the video processor 54, a CCD driver 94 for driving the CCD 59 is also provided. Moreover, at the video processor 54, a timing generator (shown as T.G in Fig. 9) 95 is also provided for receiving a synchronization signal synchronized with rotation of the rotating filter 65 from the control circuit 72 of the light source device 53 and outputting a timing signal controlling operation timing to each circuit in the video processor 54.

In the present embodiment, the subject can be observed in two modes of the ordinary observation mode and the fluorescent observation mode, and the observation mode can be set by a mode switch 96 provided at the camera head portion 58. The set observation mode is outputted to a mode switching circuit 97 provided at the video processor 54 from the mode switch 96. The mode switching circuit 97 outputs a control signal according to the set observation mode to a light control parameter circuit 98, a light control circuit 99, and the mode switching motor 70. The light control parameter circuit 98 outputs a light control parameter according to the rotating filter 65 used in the set observation mode to the light control circuit 99. That is, in the ordinary observation mode, the light control parameter according to the first filter pair 67 is outputted, while in the fluorescent observation mode, the light control parameter according to the second filter pair 68 is outputted. The light control circuit 99 controls the diaphragm device 64 so that the illumination light with an adequate brightness is irradiated to the subject based on the control signal received from the mode switching circuit 97 and the light control parameter received from the light control parameter circuit 98. The mode switching motor 70 moves the rotating filter 65 in the direction crossing the light path of the illumination light so that the filter corresponding to the set observation mode is inserted onto the light path of the illumination light. That is, in the ordinary observation mode, the rotating filter 65 is moved so that the first filter pair 67 is inserted onto the light path, while in the fluorescent observation mode, the rotating filter 65 is moved so that the second filter pair 68 is inserted onto the light path. The mode switching circuit 97 can execute gain control or control of the number of image fields, not shown, other than the above-mentioned various controls.

Action of the fluorescent observation system 51 configured as above will be described. First, the action when the ordinary observation mode is selected will be described. When the rigid endoscope 57 is arranged at a position opposite to the subject and a power supply, not shown, of the fluorescent observation system 51 is turned on, white light, which is the illumination light, is emitted from the xenon lamp 62 of the light source device 53. After the illumination light is transmitted through the heat ray cut filter 63 and the heat ray is cut off, the light is controlled to an adequate light amount by the diaphragm device 64 and enters the rotating filter 65.

When the ordinary observation mode is selected, the first filter pair 67 is inserted onto the light path of the illumination path. Thus, the incident illumination light is transmitted through the R1 filter 67r1, the G1 filter 67g1, the B1 filter 67b1, and only the light of red (R), green (G), blue (B) is filtered and emitted sequentially from the light source device 53 to the light guide 61. The illumination light of the field sequential R, G, B having entered the light guide 61 passes through the illumination window, not shown, at the tip end face of the rigid endoscope 57 and is irradiated to the subject. By being irradiated by the field sequential illumination light, diffused light, reflected light, radiated light are generated from the subject. These lights are collected by the objective optical system 73 provided at the camera head portion 58, transmitted through the excitation light cut filter 74 and the light in the wavelength region less than 470 nm is cut off, and then, an image is formed on the photoelectric conversion surface of the CCD 59 and photoelectrically converted. The time-series image capturing signal photoelectrically converted and outputted from the CCD 59 is inputted to the video processor 4 and inputted to the amplifier 81 to be amplified to an electric signal in a predetermined range (0 to 1 volt, for example). This output signal of the amplifier 81 is inputted to the process circuit 82 for processing such as correlated double sampling or noise elimination. The image capturing signal outputted from the process circuit 82 is inputted to the A/D converter 83 and converted into a digitalized image signal. The digital image signal outputted from the A/D converter 83 is inputted to the white balance circuit 84 for white balance control. The image signal outputted from the white balance circuit 84 is inputted to the 1-input/3-output selector 85. The image signal sent in the time series is separated into each color signal of R, G, B by the selector 85, sequentially inputted to the synchronization memories 86, 87, 88, and synchronized at reading out. Each signal outputted from the synchronization memories 86, 87, 88 is inputted into the image processing circuit 89 for gamma correction processing, edge enhancement processing, color tone control processing and the like. Each signal outputted from the image processing circuit 89 is inputted to the D/A converting circuits 90, 91, 92, respectively, and converted to an analog signal and outputted to the observation monitor 55. On the observation monitor 55, an endoscopic image illuminated and captured by the ordinary white light is displayed. The analog signal converted by the D/A converting circuits 90, 91, 92 is also outputted to the encoding circuit 93 other than to the observation monitor 55 and encoded. The encoded signal is outputted to the image filing device 56 and stored as a compressed image.

As shown in Fig. 10, the transmission characteristic of the first filter pair 67 is set so that the wavelength band of the light transmitted through the R1 filter 67rl and the wavelength band of the light transmitted through the G1 filter 67gl are partially overlapped, and the wavelength band of the light transmitted through the G1 filter 67gl and the wavelength band of the light transmitted through the B1 filter 67b1 are partially overlapped. Since the transmission characteristic of the first filter pair 67 is set in this way, an endoscopic image in a color tone desired by an observer and an endoscopic image in natural colors can be obtained.

Next, action in the fluorescent observation mode will be described. Cases where observation is made in the fluorescent observation mode include inspection and treatment of a cancer tissue of a bladder cancer, for example. When 5-ALA (5-Aminolevulinic acid) is delivered to a subject, which is a portion to be inspected prior to the inspection, chemical change occurs only at a tumor portion, which is a cancer tissue and Protoporphyrin IX is generated. Protoporphyrin IX has a nature that fluorescent light with the wavelength of 630 nm is excited when an excitation light with the wavelength of 417 nm is irradiated. Fluorescent observation is made using this nature.

First, when the observation mode is switched from the ordinary observation mode to the fluorescent observation mode by operating the mode switch 96 provided at the camera head portion 58, the mode switching motor 70 is operated to move the rotating filter 65 in the direction crossing the light path of the illumination light (direction shown by the arrow P in Fig. 9) so that the second filter pair 68 is inserted onto the light path. Also, the diaphragm device 64 is operated and controlled so that the illumination light with an adequate brightness is irradiated to the subject. Then, the white light emitted from the xenon lamp 62 of the light source device 53 and entering the rotating filter 65 via the heat ray cut filter 63, the diaphragm device 64 is transmitted through the excitation filters 68e1, 68e2 and the background filter 68b2 and only the excitation light with the wavelength of 385 to 435 nm and the background light with the wavelength of 475 to 525 nm are filtered and sequentially emitted from the light source device 53 to the light guide 61. The illumination light of the field sequential having entered the light guide 61 passes through the illumination window, not shown, of the tip end face of the rigid endoscope 57 and is irradiated to the subject. By being irradiated by the excitation light, fluorescent light with the wavelength of 630 nm is generated from the tumor portion, which is a cancer tissue, of the subject. Also, by being irradiated by the background light, diffused light, reflected light, radiated light are generated from the portions other than the tumor portion of the subject. These lights are collected by the objective optical system 73 provided at the camera head portion 58, transmitted through the excitation light cut filter 74 and the light in the wavelength region less than 470 nm is cut off, and then, an image is formed on the photoelectric conversion surface of the CCD 59 and photoelectrically converted. The time-series image capturing signal photoelectrically converted and outputted from the CCD 59 is inputted to the video processor 54, given the similar signal processing as the ordinary observation mode and outputted to the observation monitor 55. On the observation monitor 55, a fluorescent image illuminated and captured by the background light, which is the excitation light and the white light, is displayed. The observer can perform treatment such as incision of the cancer tissue while observing the displayed fluorescent image. The analog signal converted by the D/A converting circuits 90, 91, 92 is also outputted to the encoding circuit 93 other than to the observation monitor 55 and encoded. The encoded signal is outputted to the digital filing device 56 and stored as a compressed image.

In this way, in the fluorescent observation system 51 of the present embodiment, the second filter pair 68 is set to have a discrete spectral characteristic and transmit the field sequential light of two narrow bands, and variation in the optical brightness (transmission) of the fluorescent intensity and the intensity of the background light can be suppressed and reflected light with stable brightness and color tone can be detected all the time and moreover, contract between fluorescent light and reflected light can be improved.

### (Fourth embodiment)

Next, a fourth embodiment of the present invention will be described. The fluorescent observation system in the present embodiment has the same configuration as that of the fluorescent observation system in the third embodiment, so the same configurations are given the same reference numerals and the description will be omitted. Only action in the fluorescent observation mode, which is characteristic, will be described.

In the white light emitted from the xenon lamp 62 of the light source device 53 and entering the rotating filter 65 via the heat ray cut filter 63, the diaphragm device 64, only the excitation light with the wavelength of 385 to 435 nm and the background light with the wavelength of 475 to 525 nm are filtered and sequentially emitted from the light source device 53 to the light guide 61. The illumination light of the field sequential having entered the light guide 61 passes through the illumination window, not shown, of the tip end face of the rigid endoscope 57 and is irradiated to the subject. By being irradiated by the excitation light, fluorescent light with the wavelength of 630 nm is generated from the tumor portion, which is a cancer tissue, of the subject. Also, by being irradiated by the background light, diffused light, reflected light, radiated light are generated from the portions other than the tumor portion of the subject. These lights are collected by the objective optical system 73 provided at the camera head portion 58, transmitted through the excitation light cut filter 74 and the light in the wavelength region less than 470 nm is cut off, and then, an image is formed on the photoelectric conversion surface of the CCD 59 and photoelectrically converted. The time-series image capturing signal photoelectrically converted and outputted from the CCD 59 is inputted to the video processor 54.

The image capturing signal inputted to the video processor 54 is, after predetermined signal processing at the amplifier 81, the process circuit 82, the A/D converter 83, the white balance circuit 84, is inputted to the 1-input/3-output selector 85. The image signal sent in the time series is separated into each signal of the fluorescent signal by the excitation light transmitted through the excitation filter 68e1, the fluorescent signal by the excitation light transmitted through the excitation filter 68e2, the background light signal, sequentially inputted to the synchronization memories 86, 87, 88, and synchronized at reading out. Each signal outputted from the synchronization memories 86, 87, 88 is inputted into the image processing circuit 89 for gamma correction processing, edge enhancement processing, color tone control processing and the like. Particularly, in the color tone control processing, such a processing is applied that the fluorescent light is in the color opposite to that of the background light all the time. Each signal outputted from the image processing circuit 39 is inputted to the D/A converting circuits 90, 91, 92, respectively, and converted to an analog signal and outputted to the observation monitor 55. On the observation monitor 55, a fluorescent image illuminated and captured by the excitation light and the background light is displayed. The analog signal converted by the D/A converting circuits 90, 91, 92 is also outputted to the encoding circuit 93 other than to the observation monitor 55 and encoded. The encoded signal is outputted to the digital filing device 56 and stored as a compressed image.

In this way in the fluorescent observation system 51 of the present embodiment, by applying the color tone control processing at the image processing circuit 89 so that the fluorescent light is in the color opposite to that of the background light all the time, variation in color of the detected light (fluorescent light and reflected light of the background light) caused by variation in light wavelength transmitted through the excitation filters 68e1, 68e2 and the background filter 68b2 is corrected and the contrast between the fluorescent light and the reflected light can be further improved.

From the above embodiments, the present invention is characterized by the appended claims.

### Industrial applicability

When fluorescent light emitted from a tumor portion or the like is observed by delivering an agent to a portion to be inspected such as the tumor portion in a body, intensity of the background light is suppressed and a fluorescent image easy to be observed can be obtained.

## Claims

1. A light source device which can selectively supply visible light and excitation light for fluorescent observation to an endoscope provided with an excitation light cut filter which cuts off the excitation light,
wherein excitation light generating means for generating the excitation light is provided so that an excitation light transmission wavelength band for background illumination transmitting the excitation light is formed in a transmission wavelength region in the excitation light cut filter and a transmission light amount of the excitation light in the excitation light transmission wavelength band is more than 0% and not more than 2% of the total light amount of the excitation light.

2. The light source device according to claim 1, wherein the excitation light generating means comprises illumination light generating means for generating illumination light and a filter having the excitation light transmission wavelength band from the illumination light and generating excitation light of 2% or less of the total light amount of the excitation light.

3. The light source device according to claim 1, wherein the excitation light generating means has wavelength characteristic generating the excitation light whose intensity is small and distributed over a broad band in the excitation light transmission wavelength band.

4. A light source device which can selectively supply visible light and excitation light for fluorescent observation to an endoscope provided with an excitation light cut filter which cuts off the excitation light,
wherein excitation light generating means for generating the excitation light is provided so that, in the vicinity of cut-off wavelength in the transmission wavelength region in the excitation light cut filter, an excitation-light transmission wavelength band for background illumination of more than 0 and not more than 50 nm transmitting the excitation light is formed, and a transmission light amount of the excitation light in the excitation light transmission wavelength band is 2% or less of the total light amount of the excitation light.

5. The light source device according to claim 4, wherein the excitation light generating means has illumination light generating means for generating illumination light and a filter having the excitation light transmission wavelength band from the illumination light and generating excitation light of 2% or less of the total light amount of the excitation light.

6. The light source device according to claim 4, wherein the excitation light generating means has wavelength characteristic generating the excitation light whose intensity is small and distributed over a broad band in the excitation light transmission wavelength band.

7. A fluorescent observation system for displaying a fluorescent image which can be obtained by irradiating excitation light to a subject containing a fluorescent substance, comprising:
a light source for generating illumination light including the excitation light;
filter means arranged between the light source and the subject for alternately transmitting the excitation light and background light of a wavelength band separated from the excitation light for the illumination light emitted from the light source;
image capturing means for capturing a fluorescent image of the subject obtained by the excitation light and a background light image of the subject obtained by the background light; and
image processing means for synchronizing the fluorescent image and the background light image, applying image processing and outputting to be displayed on image display means as a fluorescent image.

8. The fluorescent observation system according to claim 7, wherein the image processing means has color tone control means for applying color-tone control to make the background light image in the color opposite to that of the fluorescent image.

9. The fluorescent observation system according to claim 7, further comprising second filter means cutting off the excitation light and transmitting the background light image and the fluorescent image between the subject and the image capturing means.

10. The fluorescent observation system according to claim 8, further comprising second filter means cutting off the excitation light and transmitting the background light image and the fluorescent image between the subject and the image capturing means.

11. A light source device which can selectively supply visible light and excitation light for fluorescent observation to an endoscope provided with an excitation light cut filter which cuts off the excitation light, comprising:
a rotating filter provided with a plurality of first filters for supplying the excitation light and a plurality of second filters for supplying background light in a wavelength band separated from the excitation light arranged in the field sequential manner.
